# EUROPEAN PATENT SPECIFICATION

(11) **EP 3 091 345 B1**
(45) Date of publication and mention of the grant of the patent: **28.04.2021**
(21) Application number: 14876256.0
(22) Date of filing: 27.01.2014
(51) Int. Cl.: G01N 1/38, G01N 1/14, A61B 10/00, B01F 7/00, B01F 7/16, B01L 3/00

(54) **METHOD AND DEVICE FOR PREPARING EXCREMENT DETECTION SOLUTION, AND DIAFILTRATION APPARATUS THEREOF**
VERFAHREN UND VORRICHTUNG ZUR HERSTELLUNG EINER LÖSUNG FÜR DEN NACHWEIS VON EXKREMENTEN UND DIAFILTRIERUNGSVORRICHTUNG DAFÜR
PROCÉDÉ ET DISPOSITIF PERMETTANT DE PRÉPARER UNE SOLUTION DE DÉTECTION D'EXCRÉMENTS, ET SON APPAREIL DE DIAFILTRATION

(30) Priority: 02.01.2014 CN 201410000964
(43) Date of publication of application: 09.11.2016
(73) Proprietor: AVE Science & Technology Co., Ltd., Changsha, Hunan 410013 (CN)
(72) Inventor: DING, Jianwen, Hunan 410013 (CN)
(74) Representative: Grünecker Patent- und Rechtsanwälte PartG mbB
(86) International application number: PCT/CN2014/071530
(87) International publication number: WO 2015/100832

(56) References cited:
- EP-A1- 1 845 357
- WO-A1-2012/150713
- CN-A- 102 998 150
- CN-A- 102 998 150
- CN-U- 201 867 313
- CN-U- 201 897 539
- CN-U- 202 158 986
- CN-U- 202 994 538
- CN-U- 203 178 092
- CN-U- 203 720 006
- CN-Y- 201 096 678
- US-A1- 2006 115 385
- US-A1- 2006 188 939

## Description

This application claims the benefit of priority from the prior Chinese Patent Application, having an application No. 201410000964.6 and an invention name "method and apparatus for preparing feces detection fluid, and dilution filter device thereof" and filed to the Patent Office of the People's Republic of China on January 02, 2014, the entire contents of which are incorporated herein.

### BACKGROUND

### Technical Field

The present invention relates to the technical field of detection devices, more particularly to a dilution filter apparatus and a method for preparing feces detection fluid, and the present invention further relates to a device for preparing feces detection fluid having the dilution filter apparatus.

### Related Art

Feces detection is one of main detection projects for examining diseases of a patient. Before the visible component detection on the feces, generally, a device for preparing the feces detection fluid is required to perform dilution, filter and sampling operations to obtain suspension for visible component detection, a specific process comprises: collecting a feces sample, placing the sample in a dilution filter apparatus (being a constituting apparatus of the device for preparing the feces detection fluid), then mounting the dilution filter apparatus containing the feces sample on a stirring sampling device (being another constituting apparatus of the device for preparing the feces detection fluid), to perform dilution, stirring, filter and sampling operations on the sample, and sending the obtained sample to a detection apparatus for detection.

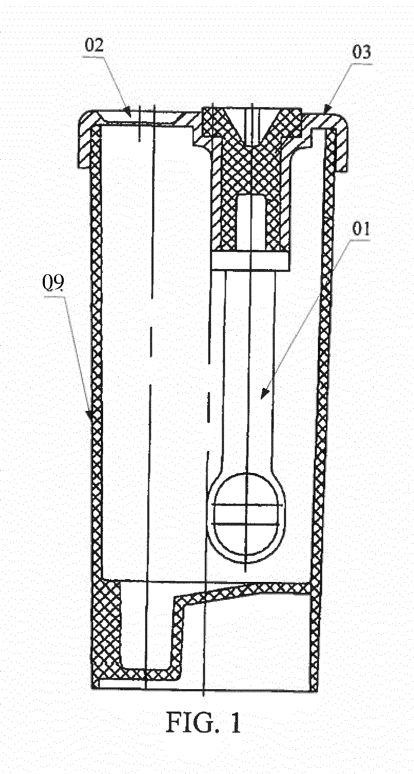

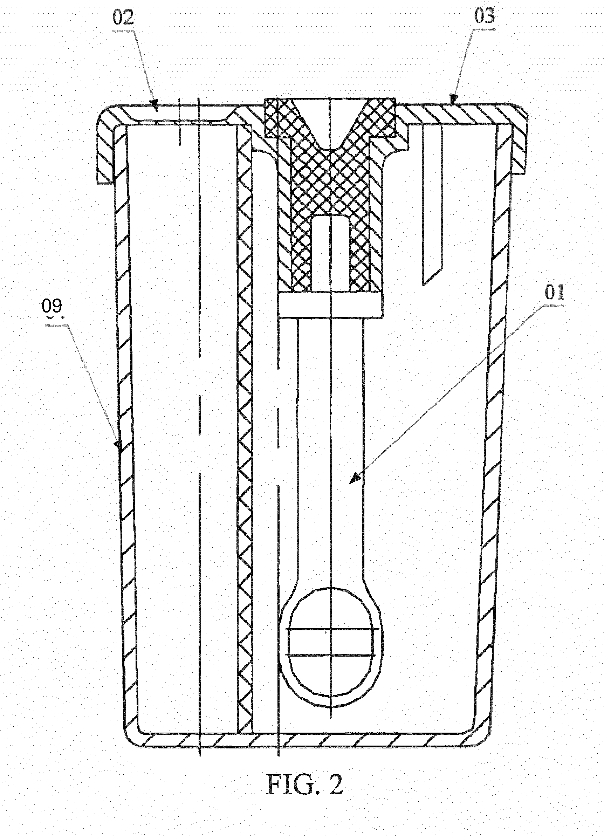

In prior art, there are many dilution filter apparatuses with different structures, but each dilution filter device comprises a sampling component 01 and an insertion hole 02, and Figs 1 and 2 show two of the many dilution filter apparatuses, wherein the sampling component 01 is a sampling spoon and is rotatably arranged in the center of a top cover 03 of the dilution filter apparatus and used for placing the feces sample in a sample tube body 04 of the dilution filter apparatus, and after diluent is injected into the sample tube body 04, the sampling component 01 rotates to stir the mixed suspension of the diluent and the feces sample; the insertion hole 02 is arranged in the top cover 03 as well but is arranged at the part of the top cover 03 close to the edge to reserve a mounting space for the sampling component 01, the insertion hole 02 is a passage for a sampling needle on the device for preparing the feces detection fluid to enter and exit the sample tube body 04, so that the sampling needle can smoothly inject the diluent into the sample tube body 01 and sucks the stirred suspension away to finish the sampling operation.

Since the sampling component 01 and the insertion hole 02 are arranged at different parts of the top cover 03, the area of the top cover 03 is large to have enough space to arrange the sampling component 01 and the insertion hole 02, and similarly, the cylindrical sample tube body 04 matched with the top cover 03 also needs a large cross section area to smoothly finish the dilution and sampling operations. However, due to the larger cross section area of the top cover 03 and the sample tube body 04, the dilution filter apparatus with above structure has larger integral size and larger occupied space, in addition, the insertion hole 02, the top cover 03 and the sample tube body 04 are not arranged concentrically, in the process of aligning the sampling needle with the insertion hole 02, the difficulty of mounting the dilution filter apparatus on the device for preparing the feces detection fluid is increased.

A dilution filter apparatus following this design principle is known e.g. from CN 201897539 U and CN202158986 U, whereby a vertical separating wall with two filter nets are provided dividing a sample tube body into a mixing chamber and a sampling chamber. The mixing chamber is provided mainly around a rotatable sampling spoon and the sampling chamber is provided below the insertion hole, through which a sampling needle can inject a diluent into the sampling chamber and sample a filtered mixed suspension away from the sampling chamber. The insertion hole and the top portion of the sampling spoon are spaced apart from each other on a top cover. The disadvantage is the complex structure and the required large space for the top cover.

An apparatus and a method for mixing specimens in a container are also known from EP 1 845 357 A1, whereby the cover needs to be removed for performing the stirring step and an additional filter needs to be applied. Another dilution filter apparatus is known from US 2006 / 0115385 A1, whereby the cover is provided with an additional cap which is hingedly coupled to the cover and can close an aperture provided in the middle of the cover.

A more space-saving dilution container is also known from WO 2012/150713 A1, whereby the the dilution container comprises a container and a top cover, which are engaged with each other through a helical thread. The top cover comprises a through hole, a supporting rod extending vertically into the lower part of the container, and a feces-sampling part attached to the lower end of the supporting rod. The supporting rod is provided with a hollow channel, the upper end of which is connected with the through hole and the lower end of which is surrounded by the feces sampling part. A sealing membrane is provided within the hollow channel to seal the container, before it is penetrated by a sampling needle, which can inject fluid into the container or sucks fluid out of it. The feces-sampling part filters the fluid during the suction operation. The disadvantage is the stirring is not possible without open the cover and the choice of filter material are very limited due to the construction restraint.

CN 102 998 150 A discloses a dilution filter apparatus with a sampling spoon rotatable in an upper section of a sample tube body. A filter net is located at the lower section of the sample tube body, so that the sample can be aspirated from the bottom of the sample tube body through rotation.

The problem to be solved is to provide a dilution filter apparatus, a corresponding device and a method for preparing feces detection fluid, whereby mixing of the suspension and the sample is improved, flow rates during the rotation mixing is reduced, the flow direction is changed and a direct impact of the large particles on the filtering nets is prevented, so that blocking of the filter nets with large sample particles is avoided.

### SUMMARY

Considering this, the present invention provides a dilution filter apparatus according to claim 1.

Preferably, in the dilution filter apparatus, an opening of the through hole located at the outside surface of the top cover is provided with a soft adhesive film for sealing the through hole.

Preferably, in the dilution filter apparatus, the inner wall of the sample tube body is provided with a plurality of protrusion ribs protruded out of the inner wall.

Preferably, in the dilution filter apparatus, one end of the sampling part away from the suspension collecting chamber is a collecting end capable of collecting a sample, and the collecting end is a three-edged rule-shaped, spoon-shaped, toothed, brush-shaped or mace-shaped collecting end.

The present invention further provides a method according to claim 6 for preparing feces detection fluid, which is using the dilution filter apparatus and comprises the following steps:
(1) collecting a feces sample to be detected by using the sampling component assembled on the top cover, then placing the sampling component attached to the feces sample into the sample tube body, and sleeving the top cover on the top end of the sample tube body;
(2) injecting diluent to the sampling part by the connecting cylinder;
(3) driving the sampling component to rotate around a self axis to cause the extending out plate arranged vertical to a plane where the filter net is located to push the suspension to rotate, wherein the extending out plate blocks in front of the filter net in a rotating direction till the feces sample and the diluent are mixed uniformly; and
(4) in a rotating stirring process, sucking the suspension entering the suspension collecting chamber by the filter net to obtain feces detection fluid.

The invention further provides a device according to claim 5.

In the dilution filter apparatus of the present invention, the through hole penetrating through the top cover is arranged in the center position of the top cover, the connecting cylinder of the sampling component is in match mounting with the through hole, after being mounted, the connecting cylinder and the through hole are in concentric connection, the connecting cylinder can freely rotate around a common axis of the connecting cylinder and the through hole, the sampling part is in concentric connection with the connecting cylinder and the through hole, and after the top cover and the sample tube body are connected, the sampling component can be totally contained in the sample tube body. When the dilution filter apparatus is used, firstly, a sample is placed in the sample tube body, then the diluent is injected into the sample tube body, then the connecting cylinder is rotated and drives the sampling part to rotate to stir suspension and enable the suspension to enter the sampling part, after the suspension is stirred, and the sampling needle enters the through hole, and penetrates through the through hole and connecting cylinder which are concentric to enter the sampling part finally and suck the suspension in the sampling part so as to finish the sampling operation.

In the dilution filter apparatus provided by the present invention, the through hole, the connecting cylinder and the sampling part which are concentrically arranged replace a sampling component and an insertion hole arranged on the top cover in an original structure, thereby realizing the dilution, stirring and sampling operation of the dilution filter apparatus by only arranging the through hole in the center of the top cover, solving the problem of large occupied area due to the fact that the sampling component and the insertion hole are both arranged on the top cover, further reducing a design size of the top cover and the sample tube body, reducing the integral size of the dilution filter apparatus and saving a space resource. In addition, due to the through hole, the connecting cylinder and the sampling part which are concentrically, a sampling hole can be directly aligned with the center of the top cover to realize the sampling operation, and the mounting difficulty of the dilution filter apparatus on the device for preparing the feces detection fluid is reduced. Again, visible components in the suspension are collected by rotating the sampling part, thereby ensuring that the visible components of the sample are collected furthest and facilitating subsequent detection. The present invention further provides a device with the above dilution filter apparatus for preparing feces detection fluid and a method for preparing feces detection fluid.

### BRIEF DESCRIPTION OF THE DRAWINGS

In order to more clearly describe the technical solution in embodiments of the present invention or prior art, the drawings used in the description of the embodiments of the present invention or prior art will be briefly introduced, obviously, the drawings in following description are only some embodiments of the present invention, and those ordinary skilled in the art cab obtain other drawings according to these drawings without any inventive labor.
FIG. 1 and FIG. 2 are structural schematic diagrams of two dilution filter apparatuses of different structures provided in prior art;
FIG. 3 is an explosive view of a dilution filter apparatus provided by an embodiment of the present invention;
FIG. 4 is a working schematic diagram of an extending out plate;
FIG. 5 is a structural schematic diagram of a sampling component;
FIG. 6 is a structure schematic diagram of the sampling component from another angle;
FIG. 7 is a structural schematic diagram of a sample tube body; and
FIG. 8 is a structural schematic diagram of a stirring sampling apparatus provided by an embodiment of the present invention.

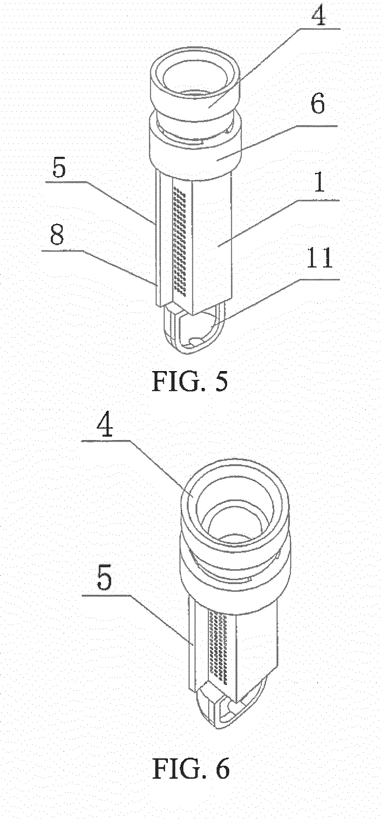

In Figs. 1-8:
Sampling component 01, top cover 02, through hole 03, connecting cylinder 4, suspension collecting chamber 5, stopping part 6, filter net 7, extending out plate 8, sample tube body 9, protrusion rib 10, collecting end 11, sampling needle 12, stirring handle 13, rack 14, vertical motion motor 15, transmission gear 16.

### DETAILED DESCRIPTION

The present invention provides a dilution filter apparatus, capable of reducing an occupied space of the dilution filter apparatus and saving a space resource.

The technical solutions in the embodiments of the present invention are clearly completely described in combination with the drawings in the embodiments of the present invention, and obviously, the described embodiments are only part of rather than all embodiments of the present invention.

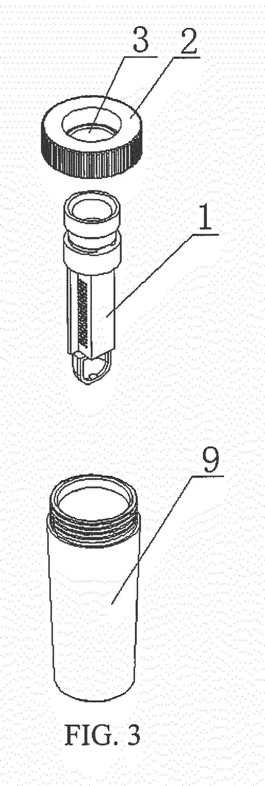

As shown in Figs 3-8, the dilution filter apparatus provided by the embodiment of the present invention comprises a sampling part component 1, a top cover 2 and a sample tube body 9, wherein
a through hole 3 is arranged in the center position of the top cover 2, and the through hole 3 can be used for being in match mounting with the sampling component 1 and can serve as a passage that a sampling needle enters/exits the dilution filter apparatus.

The sampling component 1 comprises a connecting cylinder 4 concentrically and rotatably connectable with the through hole 3 and a sampling part concentrically connectable with the connecting cylinder 4 and capable of collecting suspension; the through hole 3, the connecting cylinder 4 and a suspension colleting chamber 5 of the sampling part are concentrically arranged to cause the sampling needle to directly stretch into the sampling part to suck diluted and filtered sample suspension; the connecting cylinder 4 has an action of being matched with a stirring mechanism mentioned later, and is used for stirring the suspension and clamping the sampling component to cause the sampling component 1 no fall into the bottom of the sample tube body 9.

Correspondingly, when the dilution filter apparatus is upside down or inclined, a stopping part 6 has an action of causing the sampling component 1 not fall out of the sample tube body 9. The connecting cylinder 4 on the sampling component 1 is in detach connecting with the stopping part 6.

In the present embodiment, threads are arranged at the outer wall of an opening in the top end of the sample tube body 9, threads are arranged at the inner side wall of the top cover 2, and the sample tube body 9 and the top cover contact and rotate to realize threaded connection.

A fixing ring is arranged at the side wall of the through hole 3, the connecting cylinder 4 and the stopping part 6 are connected in a clamping manner, and when the sampling component 1 penetrate through the through hole 3, the connecting cylinder 4 and the stopping part 6 are positioned at upper and lower sides of the fixing ring.

In the dilution filter apparatus provided by the present invention, the through hole 3 penetrating through the top cover 2 is arranged in the center position of the top cover 2, the connecting cylinder 4 of the sampling component 1 is in gap match mountable with the through hole 3, so that the sapling component 1 can flexible rotate when stirring the suspension. After being mounted, the connecting cylinder 4 and the through hole 3 are in concentric connection, the connecting cylinder 4 is freely rotatable around a common axis of the connecting cylinder 4 and the through hole 3, the sampling part 1 is in concentric connection with the connecting cylinder 4 and the through hole 3, and after the top cover 2 and the sample tube body 9 are connected, the sampling component 1 can be totally contained in the sample tube body 9.

When the dilution filter apparatus is used, firstly, a sample is placed in the sample tube body 9, then the sampling needle 12 sucks diluent and pierces into a soft rubber film to inject the diluent into the sampling part, then the connecting cylinder 4 is rotated on the top cover 2 and drives the sampling part to rotate to stir suspension and enable the suspension to enter the sampling part, after the suspension is stirred, the sampling needle sucks the suspension in the sampling part so as to finish the sampling operation.

In the dilution filter apparatus provided by the present invention, the through hole 3, the connecting cylinder 4 and the sampling part which are concentrically arranged replace a sampling component 1 and an insertion hole arranged on the top cover 2 in an original structure, thereby realizing the dilution, stirring and sampling operation of the dilution filter apparatus by only arranging the through hole 3 in the center of the top cover 2, solving the problem of large occupied area due to the fact that the sampling component 1 and the insertion hole are both arranged on the top cover 2, reducing the integral size of the dilution filter apparatus and saving a space resource.

In addition, due to the through hole 3, the connecting cylinder 4 and the sampling part which are concentrically, a sampling hole can be directly aligned with the center of the top cover 2, and the mounting difficulty of the dilution filter apparatus on the device for preparing the feces detection fluid is reduced.

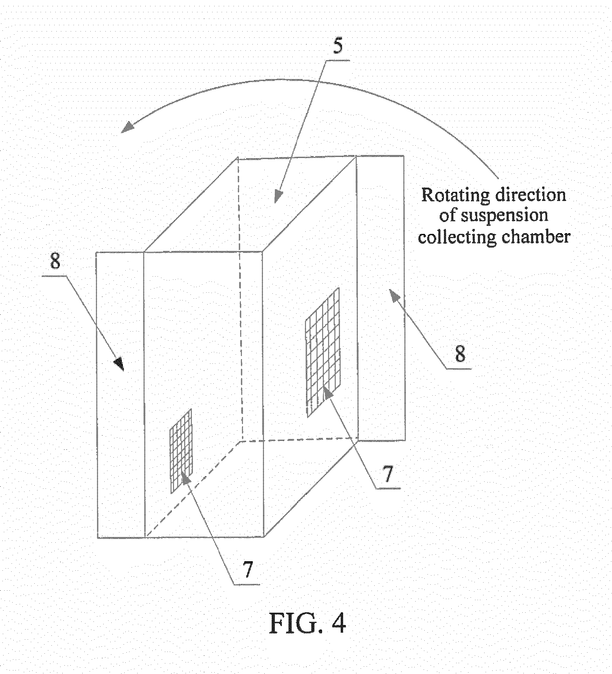

In order to further optimize the technical solution, in the dilution filter apparatus provided by the present invention, the sampling part comprises a suspension collecting chamber 5 and a plurality of dilution filter apparatus windows allowing suspension to enter the dilution filter apparatus chamber 5, wherein, the suspension collecting windows are arranged at side walls of the suspension collecting chamber 5, of which the structure is as shown in FIG. 4 and is in a right angle structure, for the suspension collecting chamber 5 in such structure, when an inner cavity thereof has a function of containing a diluted sample, the suspension collecting chamber 5 in the right angle structure also has a stirring function to match with an extending plate 8 to fully stir the suspension.

In the present invention, two suspension collecting windows are arranged preferably, each suspension collecting window is provided with a filter net 7 and the filter nets 7 arranged on the two suspension collecting windows are different in size, aperture and mounting position, if the aperture is larger, the filter nets 7 with larger areas have higher mounting position, and if the aperture is smaller, the filter nets 7 with smaller areas have lower higher mounting position. The filter nets 7 with larger aperture and area and higher mounting position facilitate particles smaller than the large aperture in the sample tube body 9 to enter the suspension collecting chamber, and due to a liquid pressure action, the filter nets 7 with smaller aperture and area and lower mounting position facilitate particles smaller than the small aperture to flow out of the suspension collecting chamber 5. During use, the particles in the suspension enter the suspension collecting chamber by the large aperture filter nets, and smaller particles and liquid leak out of the sample tube body 9 from the suspension collecting chamber through the small aperture filter nets. Due to such recycle, more particles are accumulated in the suspension collecting chamber 5 to realize an aim of improving a relevance ratio.

Preferably, the sampling part is provided with a plurality of extending out plates 8, as shown in Fig. 4-5. Since the suspension enter the suspension collecting chamber 5 by the filter nets 7 on the suspension collecting windows, when the connecting cylinder rotates, the connecting cylinder 4 drives the sampling part to rotate, therefore, a stirring action on the suspension in the sample tube body 9 is realized, and the sampling part is caused to stir the suspension which rotates along with the sampling part, and further the diluent and a feces sample are mixed uniformly.

Further, the extending out plate 8 is vertically arranged relative to a plane where the filter nets 7 are located. When the extending out plate 8 stirs the suspension under drive of the sampling part, particles in the suspension directly impact the filer nets 7, large food residue and large components in the sample may be stopped on the filter nets 7 to block the filter nets 7 and influence collection of the particles to be detected. Therefore in the present invention, a direction of the sampling part vertical to a plane where the filter net is located is provided with the extending out plate 8, the extending out plate 8 is connected with the suspension collecting chamber 5, since the rotating direction of the suspension under the stirring force action is same as that of the extending out plate 8 and the filter nets 7 are positioned behind the extending out plate 8 in the rotating direction (the rotating directions of the suspension collecting chamber 5, the suspension and extending out plate 8 are directions pointed by arrows in FIG. 4), when the extending out plate 8 impacts the suspension, the extending out plate 8 is firstly directly impacted by liquid, causing an impact force and a flow rate when the suspension flows to the suspension collecting windows behind to be reduced, thereby preventing the suspension from directly impacting the filter nets 7 to cause blockage.

The extending out plate 8 extends out of the side wall of the sampling part, and can thus achieve stirring and uniform mixing actions for the suspension. Besides, when the sampling part rotates and stirs, under the rotating and stirring actions of the extending out plate, vortexes generated by the suspension can cause effective components in the suspension to enter the suspension collecting chamber 5 and the large particles are carried away along with the vortexes and do not block the filter nets 7.

Further, an opening of the through hole 3 located at the outside surface of the top cover 2 is provided with a soft adhesive film for sealing the through hole 3 (not shown in the drawing). In the present embodiment, the opening of the through hole 3 located at the outside surface of the top cover 2 is provided with the soft adhesive film, and is used for sealing from the outside. In addition, the soft adhesive film is a film easy to pierce and causes the sampling needle to smoothly enter and exit the through hole 3.

The film in the present embodiment is a soft adhesive film preferably, and can be made of other materials meeting use requirements.

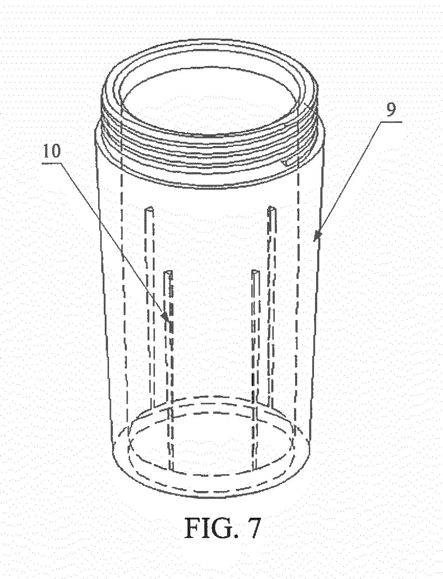

As shown in FIG. 7, the inner wall of the sample tube body 9 is provided with a plurality of protrusion ribs 10 protruded out of the inner wall. In the present embodiment, the sample tube body 9 is a containing bottle preferably, the protrusion ribs 10 are arranged at the inner wall of the sample tube body 9 and achieve a stopping action on the suspension when stirring the suspension, so that the suspension is stirred more uniformly and the stirring effect of the dilution filter apparatus provided by the present embodiment is improved.

Specifically, one end of the sampling part away from the suspension collecting chamber 5 is a collecting end 11 capable of collecting a sample, that is to say, the suspension collecting chamber 5 and suspension collecting windows of the sampling part have a suspension collecting action, the extending out plate 8 has a stirring action, the collecting end 11 has a sample collecting action, the collecting end 11 in the present embodiment is preferably a three-edged rule-shaped collecting end, the collecting end in such structure consists of three slabs in crossing connection on the same straight line, every two slabs are equal in included angle, similar to the structure of a three-edged rule, the three-edged rule-shaped collecting end can conveniently enter feces to take a pollution-free sample, when the collecting end is driven by the sampling component 1 to stir the suspension, the feces sample attached to the position is easy to smash, thereby improving a uniform mixing effect of the suspension. Of course, the collecting end 11 can be in other shapes, for example in a spoon shape, a tooth shape, a brush shape or mace shape. In the present embodiment, the shape of the collecting end 11 is not defined as long as the sample collecting and suspension stirring requirements are met.

The present embodiment further provides a method for preparing feces detection fluid, which is suitable for the dilution filter apparatus with the extending out plate 8 and comprises the following steps:
S101 assembling the sampling component 1 on the top cover 2 by a match between the connecting cylinder 4 and the stopping part 6, causing the sampling component 1 to be rotatable relative to the through hole 3, collecting a feces sample by using the sampling component 1 with the top cover 2, then placing the sampling component 1 attached to the feces sample into the sample tube body 9, sleeving the top cover 2 on the top end of the sample tube body 9, and causing the top cover 2 and the sample tube body 9 to be in threaded fastening connection;
S102 causing the sampling needle 12 pass through the connecting cylinder 4 and injecting diluent into the sampling part;
S103 extending the stirring handle 13 into the connecting cylinder 4, realizing location with the connecting cylinder 4 in a peripheral direction, rotating the stirring handle to drive the sampling component 1 to rotate around a self axis to cause the extending out plate 8 arranged vertical to a plane where the filter nets 7 are located to push the suspension to rotate, wherein in the rotating process, the filter nets 7 are positioned behind the extending plate 8 in the rotating direction, namely in the rotating direction of the sampling component 1, the extending out plate 8 always blocks in front of the filter nets 7 to prevent the suspension from directly impacting the filter nets 7 to cause blockage in the stirring process till the feces sample and the diluent are mixed uniformly; and
S104 in a rotating stirring process by the sampling needle 12, sucking the suspension entering the suspension collecting chamber 5 by the filter nets 7 to obtain feces detection fluid.

The working principles and the actions of respective parts in the method refer to the description on the working principles and the actions of respective corresponding parts in the dilution filter apparatus provided by the present embodiment and are not repeated herein.

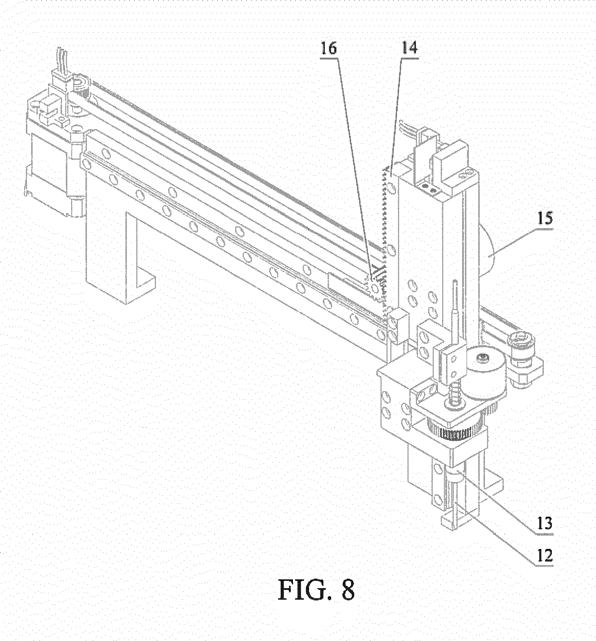

Based on the dilution filter apparatus provided in the present embodiment, an embodiment of the invention further provides a device for preparing the feces detection fluid, as shown in FIG. 8, comprising a dilution filter apparatus and a stirring sampling apparatus, wherein
the dilution filter apparatus comprises a sampling component 1, a top cover 2 and a sample tube body 9, and characterized in that a through hole 3 is formed in the center position of the top cover 2; the sampling component 1 comprises a connecting cylinder 4 concentrically and rotatably connectable with the through hole 3 and a sampling part concentrically connectable with the connecting cylinder 4 and capable of collecting suspension; the sample tube body 9 is connectable with the top cover 2 to cause the sample tube body 9 to sleeve outside the sampling component 1; and
the stirring sampling apparatus comprises a stirring mechanism, a sampling needle 12 and a vertical motion mechanism; the stirring mechanism has a cylindrical stirring handle matched with an inner hollow shape of the connecting cylinder 4; the sampling needle 12 is arranged on the vertical motion mechanism of the stirring sampling apparatus by the stirring mechanism and moves along a vertical direction under drive of the vertical motion mechanism, wherein the cylindrical stirring handle 13 of the stirring mechanism is connectable with the stirring mechanism by a fixing shaft capable of driving the stirring handle 13 to rotate, the fixing shaft has a penetrating inner cavity extending along a self axis, and the sampling needle 12 can penetrate through the penetrating inner cavity and the inner cavity of the stirring handle 13; the vertical motion mechanism is provided with a rack 14, and a vertical motion motor 15 of the vertical motion mechanism drives the vertical motion mechanism to move along a vertical direction by a transmission gear 16 meshed with the rack 14.

Respective embodiments in the present specification are described in a progressive manner, each embodiment emphasizes to explain the difference from other embodiments, and same and similar parts of respective embodiments are mutually referable.

## Claims

1. A dilution filter apparatus, comprising:
a sampling component (1), a top cover (2) and a sample tube (9),
whereby the sampling component (1) is assembled on the top cover (2);
whereby the sampling component (1) assembled on the top cover (2) is placed in the sample tube (9), and the top cover (2) is sleeved on a top end of the sample tube (9) causing the top cover (2) and the sample tube (9) to be in threaded fastening connection and the sample tube (9) is sleeved outside the sampling component (1);
whereby a through hole (3) is formed in the center position of the top cover (2);
whereby the sampling component (1) comprises a connecting cylinder (4) and a sampling part, the sampling part is concentrically connected with the connecting cylinder (4);
whereby the sampling part is configured for collecting suspension;
**characterized in that**:
the connecting cylinder (4) of the sampling component (1) is concentrically and rotatably connected with the top cover (2), so that the connecting cylinder (4), after being mounted, can freely rotate around a common axis of the connecting cylinder (4) and the through hole (3) of the top cover (2), and can drive the sampling part to rotate to stir suspension;
whereby the sampling part further comprises a suspension collecting chamber (5) and a plurality of suspension collecting windows communicated with the suspension collecting chamber (5);
whereby two suspension collecting windows are arranged at two opposite walls of the suspension collecting chamber (5) respectively, each suspension collecting window is provided with a filter net (7) and the filter nets (7) arranged on different suspension collecting windows are different in aperture;
whereby the sampling part is provided with a plate (8) extending out in a vertical direction to a plane where the filter net (7) is located for each of the filter nets (7), the plate (8) extending out is configured to prevent the suspension from directly impacting the filter nets (7) to cause blockage.

2. The dilution filter apparatus according to claim 1, wherein an opening of the through hole (3) located at the outside surface of the top cover (2) is provided with a soft adhesive film for sealing the through hole (3).

3. The dilution filter apparatus according to claim 1, wherein the inner wall of the sample tube (9) is provided with a plurality of protrusion ribs (10) protruded out of the inner wall.

4. The dilution filter apparatus according to any one of claims 1-3, wherein one end of the sampling part away from the suspension collecting chamber (5) is a collecting end (11) capable of collecting a sample, and the collecting end is a three-edged rule-shaped, spoon-shaped, toothed, brush-shaped or mace-shaped collecting end.

5. A device for preparing the feces detection fluid, comprising a dilution filter apparatus according to any one of the claims 1 - 4 and a stirring sampling apparatus, wherein
the stirring sampling apparatus comprises a stirring mechanism, a sampling needle (12) and a vertical motion mechanism; the stirring mechanism has a cylindrical stirring handle (13) matched with an inner hollow shape of the connecting cylinder (4); the sampling needle (12) is arranged on the vertical motion mechanism of the stirring sampling apparatus by the stirring mechanism and moves along a vertical direction under drive of the vertical motion mechanism, wherein the cylindrical stirring handle (13) of the stirring mechanism is connected with the stirring mechanism by a fixing shaft capable of driving the stirring handle (13) to rotate, the fixing shaft has a penetrating inner cavity extending along a self axis, and the sampling needle (12) can penetrate through the penetrating inner cavity and an inner cavity of the stirring handle (13); the vertical motion mechanism is provided with a rack (14), and a vertical motion motor (15) of the vertical motion mechanism drives the vertical motion mechanism to move along a vertical direction by a transmission gear (16) meshed with the rack (14).

6. A method for preparing feces detection fluid, **characterized by** using the dilution filter apparatus according to claim 1 and comprising the following steps:
(1) collecting a feces sample to be detected by using the sampling component (1) assembled on the top cover (2), then placing the sampling component (1) attached to the feces sample into the sample tube (9), and sleeving the top cover (2) on the top end of the sample tube (9);
(2) injecting diluent to the sampling part by the connecting cylinder (4);
(3) driving the sampling component (1) to rotate around a self axis to cause the plate (8) extending out arranged vertical to a plane where the filter net (7) is located to push the suspension to rotate, wherein the plate (8) extending out blocks in front of the filter net (7) in a rotating direction till the feces sample and the diluent are mixed uniformly; and
(4) in a rotating stirring process, sucking the suspension entering the suspension collecting chamber (5) by the filter net (7) to obtain feces detection fluid.

## Patentansprüche

1. Verdünnungsfilter-Vorrichtung, umfassend:
eine Probenentnahme-Komponente (1), eine obere Abdeckung (2) und eine Probenröhre (9),
wobei die Probenentnahme-Komponente (1) an der oberen Abdeckung (2) montiert ist;
wobei die an der oberen Abdeckung (2) montierte Probenentnahme-Komponente (1) in die Probenröhre (9) eingesetzt wird, und die obere Abdeckung (2) so auf ein oberes Ende der Probenröhre (9) aufgesetzt wird, dass dadurch die obere Abdeckung (2) und die Probenröhre (9) in Schraub-Befestigungsverbindung sind und die Probenröhre (9) die Probenentnahme-Komponente (1) von außen umhüllt,
wobei ein Durchgangsloch (3) an der Mittelposition der oberen Abdeckung (2) ausgebildet ist;
wobei die Probenentnahme-Komponente (1) einen Verbindungszylinder (4) sowie einen Probenentnahmeteil umfasst und der Probenentnahmeteil konzentrisch mit dem Verbindungszylinder (4) verbunden ist;
wobei der Probenentnahmeteil zum Aufnehmen von Suspension ausgeführt ist;
**dadurch gekennzeichnet, dass**:
der Verbindungszylinder (4) der Probenentnahme-Komponente (1) konzentrisch und drehbar so mit der oberen Abdeckung (2) verbunden ist, dass der Verbindungszylinder (4), wenn er montiert ist, frei um eine gemeinsame Achse des Verbindungszylinders (4) und des Durchgangslochs (3) der oberen Abdeckung (2) rotieren kann und den Probenentnahmeteil zum Rühren von Suspension bewegen kann;
wobei der Probenentnahmeteil des Weiteren eine Kammer (5) zum Aufnehmen von Suspension und eine Vielzahl von Fenstern zum Aufnehmen von Suspension umfasst, die mit der Kammer (5) zum Aufnehmen von Suspension in Verbindung stehen;
wobei zwei Fenster zum Aufnehmen von Suspension jeweils an zwei gegenüberliegenden Wänden der Kammer (5) zum Aufnehmen von Suspension angeordnet sind, jedes Fenster zum Aufnehmen von Suspension mit einem Filternetz (7) versehen ist und die an verschiedenen Fenstern zum Aufnehmen von Suspension angeordneten Filternetze (7) unterschiedliche Lochgröße haben;
wobei der Probenentnahmeteil für jedes der Filternetze (7) mit einer Platte (8) versehen ist, die sich in einer vertikalen Richtung nach außen zu einer Ebene erstreckt, in der sich das Filternetz (7) befindet, wobei die sich nach außen erstreckende Platte (8) so ausgeführt ist, dass sie verhindert, dass die Suspension direkt auf die Filternetze (7) auftrifft und Verstopfung bewirkt.

2. Verdünnungsfilter-Vorrichtung nach Anspruch 1, wobei eine Öffnung des Durchgangslochs (3), die sich an der Außenfläche der oberen Abdeckung (2) befindet, mit einer weichen Klebefolie zum Abdichten des Durchgangslochs (3) versehen ist.

3. Verdünnungsfilter-Vorrichtung nach Anspruch 1, wobei die Innenwand der Probenröhre (3) mit einer Vielzahl vorstehender Rippen (10) versehen ist, die von der Innenwand vorstehen.

4. Verdünnungsfilter-Vorrichtung nach einem der Ansprüche 1 - 3, wobei ein Ende des Probenentnahmeteils, das von der Kammer (5) zum Aufnehmen von Suspension entfernt ist, ein Ende (11) zum Aufnehmen ist, mit dem eine Probe aufgenommen werden kann, und das Ende zum Aufnehmen ein dreikantiges lattenförmiges, löffelförmiges, gezahntes, bürstenförmiges oder keulenförmiges Ende zum Aufnehmen ist.

5. Einrichtung zum Herstellen von Flüssigkeit zum Nachweis von Fäkalien, die eine Verdünnungsfilter-Vorrichtung nach einem der Ansprüche 1 - 4 sowie eine Rühr-Probenentnahmevorrichtung umfasst, wobei
die Rühr-Probenentnahmevorrichtung einen Rühr-Mechanismus, eine Probenentnahme-Nadel (12) sowie einen Vertikal-Bewegungsmechanismus umfasst; der Rühr-Mechanismus einen zylindrischen Rühr-Stiel (13), der an eine Innenhohlraum-Form des Verbindungszylinders (4) angepasst ist; die Probenentnahme-Nadel an dem Vertikal-Bewegungsmechanismus der Rühr-Probenentnahmevorrichtung über den Rühr-Mechanismus angeordnet ist und sich von dem Vertikal-Bewegungsmechanismus angetrieben in einer vertikalen Richtung bewegt, der zylindrische Rühr-Stiel (13) des Rühr-Mechanismus mit dem Rühr-Mechanismus über eine Befestigungswelle verbunden ist, die den Rühr-Stiel (13) drehend antreiben kann, die Befestigungswelle einen durchgängigen Innenhohlraum aufweist, der sich entlang einer Selbstachse erstreckt, und die Probenentnahme-Nadel (12) den durchgängigen Innenhohlraum und einen Innenhohlraum des Rühr-Stiels (13) durchdringen kann; der Vertikal-Bewegungsmechanismus mit einer Zahnstange (14) versehen ist, und ein Vertikal-Bewegungsmotor (15) des Vertikal-Bewegungsmechanismus den Vertikal-Bewegungsmechanismus mittels eines Übertragungs-Zahnrades (16), das mit der Zahnstange (14) kämmt, so antreibt, dass er sich in einer vertikalen Richtung bewegt.

6. Verfahren zum Herstellen von Flüssigkeit zum Nachweis von Fäkalien, **gekennzeichnet durch** Einsatz der Verdünnungsfilter-Vorrichtung nach Anspruch 1, wobei es die folgenden Schritte umfasst:
(1) Aufnehmen einer Fäkalienprobe zum Nachweis unter Verwendung der an der oberen Abdeckung (2) montierten Probenentnahme-Komponente (1), anschließendes Einbringen der Probenentnahme-Komponente (1) mit der daran haftenden Fäkalienprobe in die Probenröhre (9) und Aufsetzen der oberen Abdeckung (2) auf das obere Ende der Probenröhre (9);
(2) Einspritzen von Verdünnungsmittel in den Probenentnahmeteil über den Verbindungszylinder (4);
(3) Bewegen der Probenentnahme-Komponente (1) zur Drehung um eine eigene Achse, um zu bewirken, dass die sich nach außen erstreckende Platte (8), die senkrecht zu einer Ebene angeordnet ist, in der sich das Filternetz (7) befindet, die Suspension unter Druck in Drehung versetzt, wobei die sich nach außen erstreckende Platte (8) vor dem Filternetz (7) als Sperre in einer Drehrichtung wirkt, bis die Fäkalienprobe und das Verdünnungsmittel gleichmäßig vermischt sind; und
(4) Ansaugen der über das Filternetz (7) in die Kammer (5) zum Aufnehmen von Suspension eintretenden Suspension in einem Dreh-Rührvorgang, um Flüssigkeit zum Nachweis von Fäkalien zu gewinnen.

## Revendications

1. Appareil de dilution-filtration, comprenant :
un composant d'échantillonnage (1), un couvercle supérieur (2) et un tube d'échantillon (9),
moyennant quoi le composant d'échantillonnage (1) est assemblé sur le couvercle supérieur (2) ;
moyennant quoi le composant d'échantillonnage (1) assemblé sur le couvercle supérieur (2) est placé dans le tube d'échantillon (9), et le couvercle supérieur (2) est manchonné sur une extrémité supérieure du tube d'échantillon (9) amenant le couvercle supérieur (2) et le tube d'échantillon (9) à être en raccordement de fixation fileté et le tube d'échantillon (9) est manchonné à l'extérieur du composant d'échantillonnage (1) ;
moyennant quoi un trou traversant (3) est formé dans la position centrale du couvercle supérieur (2) ;
moyennant quoi le composant d'échantillonnage (1) comprend un cylindre de raccordement (4) et une partie d'échantillonnage, la partie d'échantillonnage est raccordée de manière concentrique au cylindre de raccordement (4) ;
moyennant quoi la partie d'échantillonnage est configurée pour collecter une suspension ;
**caractérisé en ce que** :
le cylindre de raccordement (4) du composant d'échantillonnage (1) est raccordé de manière concentrique et rotative au couvercle supérieur (2), de sorte que le cylindre de raccordement (4), après avoir été monté, puisse tourner librement autour d'un axe commun du cylindre de raccordement (3) et du trou traversant (3) du couvercle supérieur (2), et puisse entraîner la partie d'échantillonnage en rotation pour agiter la suspension ;
moyennant quoi la partie d'échantillonnage comprend en outre une chambre de collecte de suspension (5) et une pluralité de fenêtres de collecte de suspension en communication avec la chambre de collecte de suspension (5) ;
moyennant quoi deux fenêtres de collecte de suspension sont respectivement agencées au niveau de deux parois opposées de la chambre de collecte de suspension (5), chaque fenêtre de collecte de suspension est pourvue d'un filet filtrant (7) et les filets filtrants (7) agencés sur différentes fenêtres de collecte de suspension ont des ouvertures différentes ;
moyennant quoi la partie d'échantillonnage est pourvue d'une plaque (8) s'étendant vers l'extérieur dans une direction verticale vers un plan où le filet filtrant (7) est situé pour chacun des filets filtrants (7), la plaque (8) s'étendant vers l'extérieur est configurée pour empêcher la suspension de frapper directement les filets filtrants (7) pour provoquer un blocage.

2. Appareil de dilution-filtration selon la revendication 1, dans lequel une ouverture du trou traversant (3) située au niveau de la surface externe du couvercle supérieur (2) est pourvue d'un film adhésif souple pour sceller le trou traversant (3).

3. Appareil de dilution-filtration selon la revendication 1, dans lequel la paroi interne du tube d'échantillon (9) est pourvue d'une pluralité de nervures en saillie (10) faisant saillie hors de la paroi interne.

4. Appareil de dilution-filtration selon l'une quelconque des revendications 1 à 3, dans lequel une extrémité de la partie d'échantillonnage éloignée de la chambre de collecte de suspension (5) est une extrémité de collecte (11) capable de collecter un échantillon, et l'extrémité de collecte est une extrémité de collecte en forme de règle à trois bords, en forme de cuillère, dentée, en forme de brosse ou en forme de masse.

5. Dispositif pour préparer le fluide de détection de matières fécales, comprenant un appareil de dilution-filtration selon l'une quelconque des revendications 1 à 4 et un appareil d'agitation-échantillonnage,
dans lequel l'appareil d'agitation-échantillonnage comprend un mécanisme d'agitation, une aiguille d'échantillonnage (12) et un mécanisme de déplacement vertical ; le mécanisme d'agitation a une poignée d'agitation cylindrique (13) adaptée à une forme creuse interne du cylindre de raccordement (4) ; l'aiguille d'échantillonnage (12) est agencée sur le mécanisme de déplacement vertical de l'appareil d'agitation-échantillonnage par le mécanisme d'agitation et se déplace le long d'une direction verticale sous l'action du mécanisme de déplacement vertical, dans lequel la poignée d'agitation cylindrique (13) du mécanisme d'agitation est raccordée au mécanisme d'agitation par un arbre de fixation capable d'entraîner la poignée d'agitation (12) en rotation, l'arbre de fixation a une cavité interne pénétrante s'étendant le long d'un axe propre, et l'aiguille d'échantillonnage (12) peut pénétrer à travers la cavité interne pénétrante et une cavité interne de la poignée d'agitation (13) ; le mécanisme de déplacement vertical est pourvu d'une crémaillère (14), et un moteur de déplacement vertical (15) du mécanisme de déplacement vertical entraîne le mécanisme de déplacement vertical à se déplacer le long d'une direction verticale par un engrenage de transmission (16) engrené avec la crémaillère (14).

6. Procédé de préparation de fluide de détection de matières fécales, **caractérisé par** l'utilisation de l'appareil de dilution-filtration selon la revendication 1 et comprenant les étapes suivantes :
(1) collecter un échantillon de matières fécales à détecter en utilisant le composant d'échantillonnage (1) assemblé sur le couvercle supérieur (2), puis placer le composant d'échantillonnage (1) attaché à l'échantillon de matières fécales dans le tube d'échantillon (9), et manchonner le couvercle supérieur (2) sur l'extrémité supérieure du tube d'échantillon (9) ;
(2) injecter un diluant dans la partie d'échantillonnage par le cylindre de raccordement (4) ;
(3) entraîner le composant d'échantillonnage (1) en rotation autour d'un axe propre pour amener la plaque (8) s'étendant vers l'extérieur agencée verticalement par rapport à un plan où le filet filtrant (7) est situé à pousser la suspension à tourner, où la plaque (8) s'étendant vers l'extérieur se bloque devant le filet filtrant (7) dans une direction de rotation jusqu'à ce que l'échantillon de matières fécales et le diluant soient mélangés uniformément ; et
(4) dans un processus d'agitation rotative, aspirer la suspension entrant dans la chambre de collecte de suspension (5) par le filet filtrant (7) pour obtenir un fluide de détection de matières fécales.
